# FASCICULE DE BREVET EUROPEEN

(11) **EP 0 567 385 B1**
(45) Date de publication et mention de la délivrance du brevet: **02.09.1998**
(21) Numéro de dépôt: 93401010.9
(22) Date de dépôt: 19.04.1993
(51) Int. Cl.: A61B 1/04, A61B 1/24

(54) **Dispositif d'acquisition d'images d'un objet**
Objektbilderfassungsvorrichtung
Image acquisition device for an object

(30) Priorité: 21.04.1992 FR 9204826
(43) Date de publication de la demande: 27.10.1993
(73) Titulaire: Inglese, Jean Marc, F-78112 Fourqueux (FR)
(72) Inventeur: Inglese, Jean Marc, F-78112 Fourqueux (FR)
(74) Mandataire: Beauchamps, Georges

(56) Documents cités:
- EP-A- 280 823
- WO-A-89/11252
- DE-A- 3 411 366
- DE-A- 3 718 603

## Description

La présente invention concerne un dispositif d'acquisition d'images.

L'art antérieur faisait appel à des capteurs de lumière qui pouvaient être soit monochromes, soit couleurs. Dans le cas des capteurs monochromatiques, et dans le but de prendre des images en couleurs, il était effectué une analyse trichromatique à partir des couleurs fondamentales, obtenues par commutation mécanique de filtres colorés devant une source de lumière blanche. Dans le cas de capteurs couleurs, la lumière blanche était directement utilisée. Or dans le premier cas, les commutateurs mécaniques présentaient des inconvénients dus à leur consommation, leur encombrement et leur temps de réponse, et dans le deuxième cas les capteurs couleurs offraient une définition d'image et une sensibilité moindre.

Aussi, la présente invention a-t-elle pour objet de réaliser un dispositif d'acquisition d'images en couleurs, faisant appel à une technique d'éclairement particulièrement simple et de faible consommation électrique, associée à un capteur de lumièce monochromatique à grande sensibilité lumineuse et haute définition.

Pour atteindre ce but, le dispositif d'acquisition d'images selon la présente invention comprend les caractéristiques énoncées dans la revendication 1.

Un tel dispositif d'acquisition ne découle pas de façon évidente de l'état de la technique.

Par le document DE 34 113 66 A1 est seulement connu un dispositif d'acquisition d'images qui utilise, comme source de lumière, des diodes électroluminescentes susceptibles d'émettre des lumières spectrales différentes. Ce dispositif est conçu pour pouvoir faire une analyse spectrale de l'image mais non pas pour produire des images en couleurs. Ainsi le document n'aborde pas le problème du capteur à utiliser, ce qui constitue pourtant le problème capital qui était à résoudre. Ce document ne mentionne même pas la possibilité d'utiliser, comme capteur, un dispositif à transfert de charge.

Le document EP 0 280 823 A1 concerne une caméra vidéo dentaire qui utiliser comme capteur un dispositif à transfert de charge, mais pour réaliser des images monochromes. Le problème que l'invention résoud ne s'oppose donc pas dans ce document.

D'autres caractéristiques de l'invention font les objets de revendications dépendantes.

L'invention sera mieux comprise, et d'autres buts, caractéristiques, détails et avantages de celle-ci apparaîtront plus clairement au cours de la description explicative qui va suivre faite en référence aux dessins schématiques annexés donnés uniquement à titre d'exemple illustrant un mode de réalisation de l'invention et dans lequel :
La figure 1 présente sous forme d'un schéma bloc, un dispositif d'acquisition d'images selon la figure 1.
La figure 2 montre un premier exemple de réalisation du dispositif d'acquisition d'images selon la figure 1.
Les figures 3a et 3b représentant deux spectres de lumière émis par une source d'éclairage selon la présente invention.
La figure 4 montre en un diagramme illustrant les différentes phases de fonctionnement d'un capteur de lumière selon l'invention et en a, b et c les chronogrammes d'émission des lumières des couleurs respectivement bleue, verte et rouge.
La figure 5 est une vue schématique d'une source lumineuse à diode électroluminescente du type LED.
La figure 6 est une vue schématique d'un deuxième exemple de réalisation d'un dispositif d'acquisition d'images selon la figure 1.
La figure 7 illustre sous forme d'un schéma bloc un exemple de réalisation du dispositif de traitement des images en provenance du capteur de lumière selon l'invention.

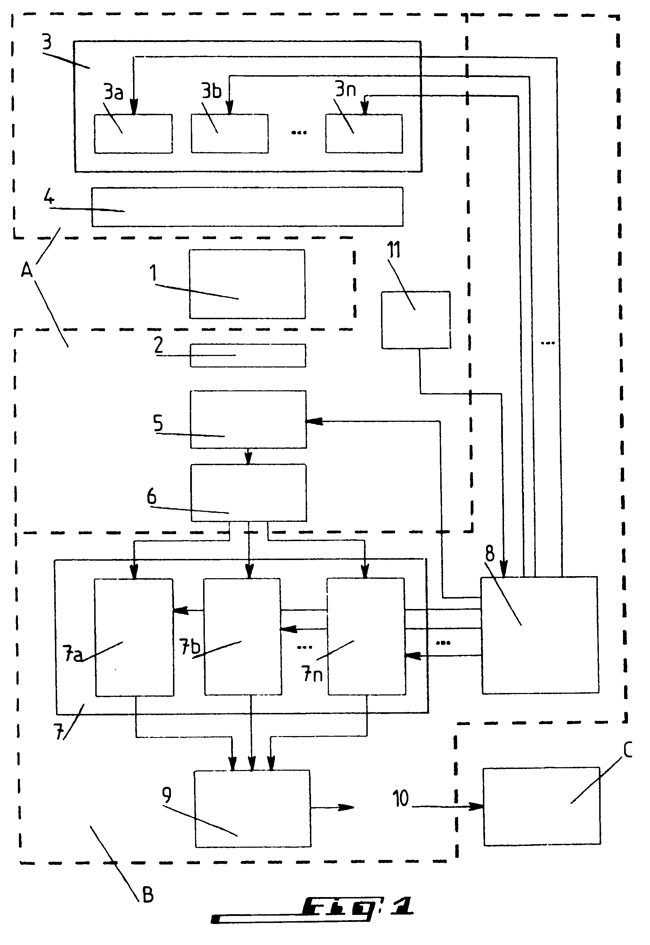

Selon la figure 1, un dispositif selon l'invention est constitué de deux sous-ensembles (A) et (B) correspondant respectivement aux fonctions d'acquisition d'images et de traitement du signal. Le sous-ensemble (A) comprend la source d'éclairage (3), les dispositifs optiques (2) et (4), le capteur de lumière (5), et optionnellement les commandes (11) d'utilisation. Le sous-ensemble (B) comprend les moyens électroniques de contrôle (8) et les moyens électroniques de stockage (7) et de reconstitution des signaux standards (9). Le sous ensemble (A) pourra, par exemple, être intégré dans une seule unité à main, tandis que le sous-ensemble (B) pourra, par exemple, être placé à distance du sujet (1).

L'image du sujet (1) est renvoyée, au travers du dispositif optique (2) vers le capteur de lumière (5). Le dispositif optique (2) pourra être constitué, par exemple, d'un objectif à lentilles associé à des miroirs, ou, dans le cas par exemple d'une application endoscopique, d'un dispositif de transfert d'images de type, par exemple, à fibres optiques.

Le capteur de lumière (5) sera choisi de type à transfert de charges (connu également sous le nom de CCD ou Charge Coupled Device) monochromatique. Le choix de la technologie CCD procède de la grande sensibilité lumineuse de ces capteurs. Le choix d'un capteur monochrome repose sur deux considérations qui sont la résolution optique à taille égale et une sensibilité lumineuse meilleures que celles des capteurs couleurs. En effet, un capteur CCD couleurs est identique à un capteur monochrome, à ceci près que devant chaque élément sensible, il a été placé un minuscule filtre coloré. Ces filtres sont choisis parmi des couleurs permettant de reconstituer une image colorée à l'aspect naturel, et associés par trois (filtres rouge, vert, bleu) ou par quatre (filtres jaune, vert, magenta, cyan). Un capteur monochrome possède donc, en raison de l'absence de filtres, une meilleure sensibilité lumineuse, due à l'absence d'absorption par le filtre, et une résolution optique meilleure, due au fait que tous les éléments sensibles sont utilisés simultanément pour une couleur donnée.

Bien sûr, si l'on désire reconstituer une image colorée, un analyse de couleurs devra être accomplie. Elle s'effectuera pour chacune des couleurs fondamentales retenues pour la source d'éclairement à raison d'une couleur à la fois.

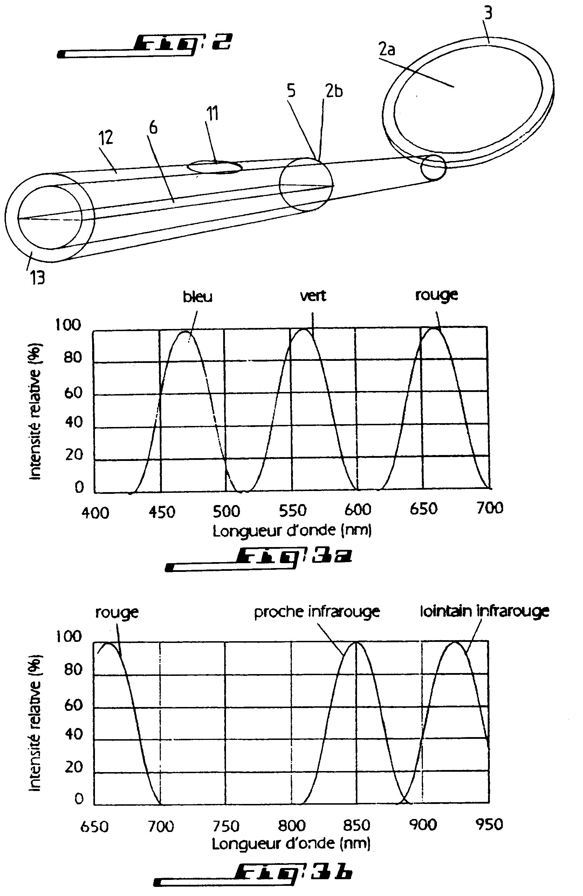

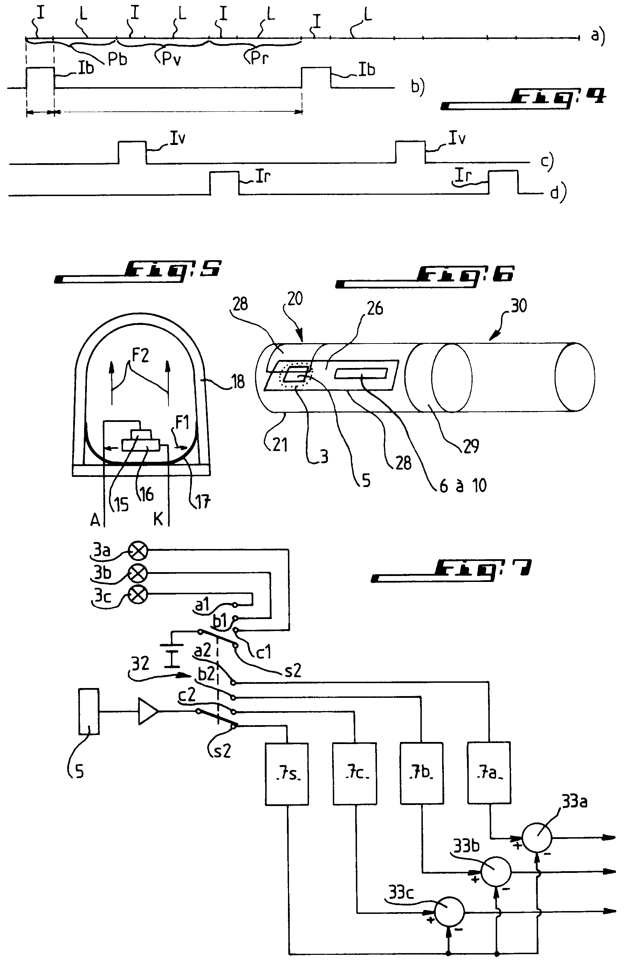

La source d'éclairement (3) sera constituée par un certain nombre de sources fondamentales de lumière de façon à reconstituer un aspect naturel des images colorées. Deux exemples de reconstitution de la lumière naturelle sont fournis par la figure 3. Le premier exemple, illustré par la figure 3a, concerne des acquisitions d'images en couleurs visibles. Il met en oeuvre un spectre de trois couleurs constitué par les couleurs fondamentales rouge, verte et bleue. Le second exemple, illustré par la figure 3b, concerne l'acquisition d'images dans la gamme infrarouge. Il met en oeuvre un spectre de trois couleurs constitué par les couleurs fondamentales rouge, proche infrarouge et lointoin infrarouge.

Les sources fondamentales de lumière seront choisies de façon à présenter un spectre d'émission aussi pur et aussi proche des couleurs fondamentales idéales que possible. Par ailleurs, ces sources devront présenter un temps de réponse très faible puisque l'analyse des couleurs s'effectuera séquentiellement. Enfin, elles devront présenter des niveaux d'émission lumineuse compatibles avec la sensibilité du capteur de lumière utilisé.

On choisira avantageusement des sources de lumière faisant appel à des émetteurs de lumière de type semi-conducteur, par exemple des diodes électroluminescentes (également connues sous le nom de LED ou Light Emitting Diodes) ou par exemple des diodes LASER, dans le cas par exemple de l'infrarouge.

En plus des avantages cités ci-dessus, les émetteurs à semi-conducteurs présentent l'intérêt d'accepter des taux de surmodulation, dans le cas d'une utilisation de type impulsionnel, ce qui sera le cas du fait de l'analyse séquentielle des couleurs, entraînant de ce fait des puissances d'émission lumineuses avantageuses.

Une autre caractéristique fondamentale des sources de lumière à semi-conducteurs est, lors du fonctionnement en lumière visible, de ne pas émettre, à la différence des autres sources de lumières dites "chaudes" telles que par exemple les ampoules à halogènes, une quantité de rayonnement infrarouge indésirable, à moins bien entendu d'être choisies spécifiquement pour cela dans le cadre d'une application déterminée. A cet effet, les capteurs à transfert de charge sont souvent munis d'un filtre qui ne laisse pas passer le rayonnement infrarouge. Dans le cas présent, ce filtre pourra donc être supprimé, ce qui entraînera une augmentation considérable de la sensibilité lumineuse du capteur de lumière (5).

L'invention permet de tenir compte des puissances d'émission des diodes électroluminiscentes du type LED, qui pourraient être différentes d'une couleur à l'autre et par exemple décroître du rouge au bleu pour un même courant d'activation des diodes, et aussi des différences de sensibilité aux couleurs du capteur 5.

Pour compenser ces différences, on pourrait augmenter en conséquence le courant d'activation des diodes ou leur nombre. Dans le premier cas, en raison du faible rapport cyclique des signaux lumineux émis par chaque diode et ainsi du mode de fonctionnement impulsionnel de celle-ci, on peut augmenter le courant d'activation sans craindre une détérioration des diodes. Même des fortes surmodulations sont ainsi possibles.

La figure 4 illustre le faible rapport cyclique de l'émission de lumière des diodes. En a, cette figure montre pour chaque couleur bleue b, verte v et rouge r les deux phases du capteur (5), à savoir la phase d'acquisition par intégration I et la phase de lecture L. Etant donné que par exemple la diode bleue n'émet que pendant le bref intervalle d'intégration I de la période bleue Pb et est hors service pendant des périodes de lumière vert Pv et rouge Pr, le rapport cyclique peut avoir une valeur très faible de par exemple 15%.

Bien entendu, les intensités lumineuses relatives des sources fondamentales de lumière pourront être ajustées pour tenir compte de la non-linéarité du diagramme de réponse spectral du capteur de lumière (5).

La figure 1 donne un exemple d'association d'un nombre n de sources fondamentales (3a), (3b), ...(3n). Chaque source fondamentale sera constituée par un ou plusieurs émetteurs à semi-conducteurs du même spectre d'émission.

On pourra optionnellement associer à ces sources fondamentales un dispositif optique (4) afin de régler leur diagramme spatial de rayonnement. Ce dispositif optique (4) pourra être constitué, par exemple, d'un objectif à lentilles associé à des miroirs, ou, dans le cas par exemple d'une application endoscopique, d'un dispositif de transfert d'images de type, par exemple, à fibres optiques.

La figure 5 montre de façon schématique un dispositif émetteur de lumière à diode LED. Sur cette figure la référence 15 désigne la puce où l'élément semi-conducteur, par exemple en silicium, qui est relié à la borne d'anode A, la référence 16 le substrat formant la cathode K et sur laquelle est posée la puce de silicium 15, et la référence 17 un réflecteur pour orienter la lumière produite indiquée par des flèches F1 sous forme d'un faisceau F2 qui sera émis à travers l'optique indiqué en 18 et faisant partie du boîtier du dispositif.

Au lieu de placer sur le substrat 16 un seul élément semi-conducteur 15, on pourrait y poser plusieurs éléments répartis de façon à donner un éclairage uniforme de l'objet dont l'image doit être prise et reproduite.

Le déclenchement de chacune des sources fondamentales sera commandé par les moyens électroniques de contrôle (8). En même temps que l'allumage de l'une des trois sources fondamentales, le capteur de lumière (5) sera mis en mode intégration, c'est-à-dire qu'il effectuera la conversion de l'énergie lumineuse reçue en charge électrique dans chacun de ses éléments sensibles. Les moyens électroniques de contrôle (8) commanderont ensuite l'extinction de la source fondamentale de lumière, et le passage du capteur de lumière (5) en mode lecture, au cours duquel les charges électriques accumulées pendant l'intégration seront disponibles sous forme de signaux de sortie, qui pourront être mis en forme par des moyens électroniques de mise en forme de signaux électriques (6).

Les signaux mis en forme seront reçus dans l'une des pages des moyens électroniques de stockage (7), qui seront chacune capable de conserver au moins une fois l'ensemble de la trame du capteur de lumière (5). Bien entendu, ces moyens électroniques de stockage seront organisés de façon à offrir autant de pages (7a), (7b),...(7n) que de couleurs fondamentales retenues.

Les moyens électroniques de contrôle (8) effectueront alors la même séquence d'opération pour la source fondamentale de lumière suivante, et ainsi de suite.

Lorsque toutes les sources fondamentales de lumière auront été séquentiellement activées, l'ensemble des moyens de stockage (7) contiendra les trames correspondant à l'image de l'objet (1) éclairé par toutes les couleurs fondamentales à raison d'une couleur par page. Pendant que le cycle décrit dans les paragraphes ci-dessus va redémarrer, les moyens de stockage pourront, à l'aide d'un dispositif de multiplexage dont ils seront munis, restituer en parallèle les trames du cycle précédent et les fournir, sous forme de signaux, aux moyens électroniques (9) qui transformeront ces signaux en de seconds signaux (10) compatibles avec les standards de visualisation.

Bien entendu, pour que cette transformation soit possible, il faudra que les cycles d'acquisition d'images en couleurs fondamentales soient plus rapides que le cycle de transfert d'une image couleurs aux moyens desdits seconds signaux. Le rapport entre la durée du cycle d'acquisition d'une trame pour une couleur fondamentale et la durée cycle de transmission d'image au moyen de signaux standards de visualisation sera égal au nombre de couleurs fondamentales utilisées par la source d'éclairement.

Ces signaux seront avantageusement affichés par des moyens (C) constitués, par exemple, par des écrans de télévision ou des dispositifs de stockage permanents d'images. Ces moyens sont connus en eux-mêmes et ne seront donc pas décrits.

Bien sûr, du fait que l'ensemble des trames correspondant à l'image du sujet (1) sera à tout instant conservé par les moyens électroniques de stockage (7), l'utilisateur pourra suspendre le cycle de prise d'image au travers d'un moyen de commande (11) pouvant être constitué, par exemple, d'un bouton situé, par exemple, sur l'unité englobant le capteur de lumière. Les moyens électroniques de contrôle suspendront alors le cycle d'acquisition de trames, tout en maintenant activée la fonction de conversion des moyens électroniques (9). On aura donc un effet de gel d'images.

L'ensemble des moyens et dispositifs ci-dessus faisant appel à des technologies à haute densité d'intégration et à faible consommation électrique, le dispositif d'acquisition d'images utilisant une source de lumière à semi-conducteurs objet de la présente description pourra avantageusement être intégré, pour tout ou partie, dans une seule unité de faible dimension, optionnellement étanche et stérilisable, qui pourra s'accommoder par exemple de dispositifs de visualisation portatifs alimentés par une source d'énergie autonome.

Un exemple de réalisation, appliqué au domaine dentaire, est fourni par la figure 2. Dans cet exemple, le sous-ensemble (A) a été intégré dans une seule unité ayant la forme d'un outil connu sous le nom de miroir du dentiste.

Les émetteurs à semi-conducteurs (3) sont disposés tout autour du miroir (2a) proprement dit. Celui-ci renvoie l'image du sujet vers le capteur de lumière (5) situé au bout de la poignée de maintien (12) au travers d'une lentille (2b). La poignée de maintien contient les moyens électroniques de mise en forme (6), et un connecteur (13) destiné à recevoir un câble de transmission de signaux.

L'utilisateur dispose d'un bouton poussoir (11) destiné à activer la fonction de gel d'images.

L'ensemble des moyens du sous-ensemble (A) est donc, dans cet exemple donné à titre illustratif mais nullement limitatif, intégré dans un seul instrument. Celui-ci pourra être rendu étanche et être stérilisé de la même manière que le miroir du dentiste traditionnel.

La figure 6 montre, de façon schématique, un autre exemple de réalisation d'un dispositif d'acquisition d'images notamment en couleurs selon l'invention. Sur cette figure, le numéro de référence 20 désigne une pièce qui comprend une enveloppe par exemple cylindrique 21 qui enferme hermétiquement les parties actives du dispositif d'acquisition d'images, telles que les diodes LED 3, le capteur du type CCD 5, le dispositif éléctronique de contrôle 6 à 10, ces parties étant disposées sur une plaquette de circuit intégré 26. L'enveloppe 21 comporte une fenêtre 28 d'émission et de réception de la lumière d'éclairage et d'image de l'objet à représenter, cette fenêtre comprenant l'optique précitée. Une extrémité indiquée en 29 est configurée en moyen de connection à une partie 30 du dispositif, qui est réalisée sous forme d'un manchon ou d'une poignée. La connection pourrait se faire par filetage.

L'enveloppe 21 est réalisée en un matériau résistant aux contraintes thermiques, ce qui permet, après sa séparation du manchon 30 et après chaque utilisation, une stérilisation de la pièce 20 dans un autoclave par exemple à une température de 120° pendant une heure. En raison des diodes LED comme sources lumière et d'un capteur du type CCD, la pièce 20 peut avoir de très faible dimensions et avoir par exemple un diamètre de 5 millimètres et une longueur de 15 millimètres.

La figure 7 montre une réalisation avantageuse du dispositif d'acquisition d'images, qui permet d'éliminer l'influence de la lumière ambiante qui est préjudiciable à la qualité de l'image reproduite. Le dispositif selon la figure 7 comprend trois sources élémentaires émettrices de lumière 3a à 3c. Le capteur 5 est adapté pour prendre successivement quatre images, une image pour chaque couleur et une image prise à la lumière ambiante. Les images en couleurs sont séparément stockées dans des mémoires 7a à 7c et l'image prise à la lumière ambiante est stockée dans une mémoire 7s. Un commutateur bipolaire 32 à 4 positions a1 à c1 et s et a2 à c2 et s2 est prévu pour relier le capteur 5 aux différentes mémoires de stockage des images individuelles, en synchronisme avec l'activation des sources de lumière élementaires. A chaque mémoire 7a à 7c est associé un dispositif 33a à 33c qui a pour fonction de soustraire lors de l'établissement de l'image résultant des trois images élémentaires monochromes stockées dans les mémoires 7a à 7c, une image de l'objet prise à la lumière ambiante et stockée dans la mémoire 7s, pour éliminer l'influence de la lumière ambiante.

Il ressort de la description de l'invention, qui vient d'être faite, que le dispositif d'acquisition d'images selon la l'invention présente de nombreux avantages découlant notamment de l'utilisation de diodes électroluminescente ou à laser comme source de lumière, en combinaison avec un dispositif capteur de lumière du type monochrome et à transfert de charge. Ainsi le dispositif d'acquisition d'images selon l'invention permet de supprimer des filtres réjecteurs des rayons infrarouges et assure une réalisation d'un dispositif de faibles dimensions. L'utilisation d'émetteurs de lumière à semi-conducteurs procure une lumière stable et calibrée, ce qui permet de procéder à des analyses calorimétriques précises en se servant du dispositif par exemple à des fins diagnostiques et pour la prise d'une teinte lors de l'élaboration d'une prothèse dentaire.

## Revendications

1. Dispositif d'acquisition d'images en couleur d'un objet, comportant une source (3) pour émettre successivement des lumières monochromes de couleurs différentes, un capteur (5) de la lumière représentative de l'image dudit objet et des moyens de traitement (B) des signaux en provenance du capteur pour reproduire une image en couleurs à partir des images en monochrome produites par le capteur (5), par analyse séquentielle de celles-ci, la source (3) comprenant, pour chaque lumière monochrome, au moins un émetteur de lumière à semi-conducteur (3a-3c) telle qu'une diode électroluminescente ou laser, des moyens (8) étant prévus pour activer successivement les émetteurs de lumière (3a-3c) de couleurs différentes, et le capteur de lumière (5) étant du type à transfert de charge monochrome.

2. Dispositif d'acquisition d'images selon la revendication 1, caractérisé en ce que la source (3) comprend des émetteurs (3a-3c) de lumières de couleurs fondamentales visibles ou invisibles, chaque émetteur étant formé par au moins une diode électroluminescente émettant de la lumière de la fréquence lumineuse fondamentale correspondante.

3. Dispositif d'acquisition d'images selon l'une des revendications 1 à 2, caractérisé en ce que les diodes électroluminescentes (3a-3c) sont des diodes surmodulables en mode de fonctionnement impulsionnel et que le rapport cyclique de l'activation des diodes lors d'un cycle d'émission est faible, de façon à permettre par une variation de la puissance de la lumière émise, une compensation des différences de puissance de lumière de couleurs différentes émises et des différences de sensibilités aux lumières de couleurs différentes du capteur.

4. Dispositif selon l'une des revendications 2 ou 3, caractérisé en ce que chaque émetteur (3a à 3c) comprend une pluralité de diodes électroluminescentes dont le nombre est choisi pour compenser les différences de puissance d'émission et de sensibilité aux lumières de couleurs différentes du capteur (5).

5. Dispositif selon l'une des revendications précédentes, caractérisé en ce que le capteur de lumière monochrome (5) présente une grande sensibilité pour des fréquences lumineuses fondamentales de la source d'éclairement (3).

6. Dispositif selon l'une des revendications précédentes, caractérisé en ce que la source d'éclairement (3) comprend un dispositif optique formé par des lentilles optiques, des miroirs ou fibres optiques, formant des moyens (4) de modification du diagramme de rayonnement de la source d'éclairement et d'adaptation de l'image de l'objet à représenter aux dimensions du capteur de lumière (5).

7. Dispositif selon l'une des revendications 2 à 5, caractérisé en ce que, pour former une source de lumière comportant plusieurs émetteurs de lumière, les éléments semi-conducteurs (5) d'au moins certains de ces émetteurs de lumière sont disposés sur un même substrat (16) et en ce qu'un dispositif optique (18) commun est associé à ces émetteurs de lumière.

8. Dispositif selon l'une des revendications 1 à 6, caractérisé en ce qu'il comprend un module de contrôle (8) du fonctionnement du capteur de lumière (5), qui commande de façon synchrone l'activation des émetteurs (3a à 3c) de la source d'éclairement (3) et un module de stockage (7) organisé en pages (7a, 7b-7n), qui reçoivent chacune de façon successive les signaux de sortie du capteur de lumière (5) correspondant à l'objet éclairé par l'une des couleurs fondamentales de la source de lumière (3), et un module de conversion (9) qui transforme les signaux de capteur de lumière (5) en signaux compatibles avec le module de stockage (7) et les signaux du module de stockage (7) en des signaux (10) compatibles avec les standards de visualisation d'images couleurs.

9. Dispositif selon l'une des revendications précédentes, caractérisé en ce qu'il comprend des moyens de commande (11) pour geler une image.

10. Dispositif selon l'une des revendications précédentes, caractérisé en ce que la source de lumière (3), le capteur de lumière (5) et avantageusement les moyens de traitement des signaux sont intégrés dans une seule unité de faible dimension, le cas échéant étanche et stérilisable.

11. Dispositif selon l'une des revendications 1 à 9, caractérisé en ce que, pour éliminer l'influence de la lumière ambiante, il comprend des moyens pour prendre une image de l'objet à la lumière ambiante et des moyens (33) de soustraction de cette image des images monochromes avant la reproduction de l'image en couleurs résultantes, de façon à éliminer la lumière ambiante dans les images monochromes.

## Claims

1. Device for the acquisition of colour images of an object, comprising a source (3) for successively emitting monochromic lights with different colours, a sensor (5) for the light representative of the image of the said object and means (B) for processing the signals coming from the sensor for reproducing a colour image from monochromous images produced by the sensor (5) by a sequential analysis of the images, the source (3) comprising for each monochromic light, at least one light emitter with a semi-conductor (3a-3c) such as an electroluminescent diode or laser, means (8) being provided for successively activating the emitters (3a-3c) of light with different colours and the light sensor (5) being of the monochromous charge transfer type.

2. Device for the acquisition of images according to claim 1, characterized in that the source (3) comprises emitters (3a-3c) of lights with visible or invisible primary colours, each emitter being formed by at least one electroluminescent diode emitting the light of the corresponding fundamental light frequency.

3. Device for the acquisition of images according to one of claims 1 to 2, characterized in that the electroluminescent diodes (3a-3c) are diodes overmodulatable in the impulsing operating mode and in that the cyclic ratio of the activation of the diodes during an emission cycle is small so as to permit by a variation of the power of the emitted light, a compensation for the emitted power differences of light with different colours and for the differences of sensitivities to the lights with different colours from the sensor.

4. Device according to one of claims 2 or 3, characterized in that each emitter (3a to 3c) comprises a plurality of electroluminescent diodes the number of which is chosen to compensate for the differences of emission power and of sensitivity to lights with different colours from the sensor (5).

5. Device according to one of the foregoing claims, characterized in that the monochromic light sensor (5) exhibits a great sensitivity for fundamental light frequencies of the lighting source (3).

6. Device according to one of the foregoing claims, characterized in that the lighting source (3) comprises an optical device formed by optical lenses, mirrors or optical fibres, forming means (4) for the modification of the radiation diagram of the lighting source and for the adaptation of the image of the object to be represented to the dimensions of the light sensor (5).

7. Device according to one of claims 2 to 5, characterized in that to form a light source comprising several light emitters, the semi-conductor elements (5) of at least some of these light emitters are arranged on a same substrate (16) and in that a common optical device (18) is associated with these light emitters.

8. Device according to one of claims 1 to 6, characterized in that it comprises a module (8) for the control of the operation of the light sensor (5), which controls in a synchronous fashion the activation of the emitters (3a to 3c) of the lighting source (3) and a storage module (7) organized in pages (7a, 7b-7n), which receive each one in a successive manner the output signals from the light sensor (5) corresponding to the object illuminated by one of the primary colours of the light source (3) and a conversion module (9) which converts the signals from the light sensor (5) into signals compatible with the storage module (7) and the signals from the storage module (7) into signals (10) compatible with the standards for the visualization of colour images.

9. Device according to one of the foregoing claims, characterized in that it comprises control means (11) for freezing an image.

10. Device according to one of the foregoing claims, characterized in that the light source (3), the light sensor (5) and advantageously the signal processing means are integrated into one single unit of small size which if need be is sealed and sterilizable.

11. Device according to one of claims 1 to 9, characterized in that for removing the influence of the ambient light, it comprises means for taking an image of the object in the ambient light and means (33) of subtraction of this image from the monochromic images prior to the reproduction of the images with resulting colours, so as to remove the ambient light in the monochromic images.

## Patentansprüche

1. Vorrichtung zur Erfassung von farbigen Bildern eines Gegenstandes, mit einer Quelle (3), um einfarbige Lichter mit unterschiedlichen Farben aufeinanderfolgend auszustrahlen, einem Sensor (5) für das das Bild des besagten Gegenstandes darstellende Licht und Mitteln (B) zur Verarbeitung der von dem Sensor kommenden Signale, um ein mehrfarbiges Bild ausgehend von den durch den Sensor (5) erzeugten einfarbigen Bildern durch Sequentiellanalyse derselben wiederzugeben, wobei die Quelle (3) für jedes einfarbige Licht wenigstens einen Halbleiterlichtaussender (3a-3c), wie eine Elektrolumineszensdiode oder Laser umfasst, wobei Mittel (8) vorgesehen sind, um die Aussender (3a-3c) des Lichtes mit unterschiedlichen Farben aufeinanderfolgend zu aktivieren und wobei der Lichtsensor (5) der Gattung mit einfarbiger Ladungsübertragung ist.

2. Vorrichtung zur Erfassung von Bildern gemäß Anspruch 1, dadurch gekennzeichnet, daß die Quelle (3) Ausstrahler (3a-3c) für sichtbare oder unsichtbare Lichter mit Grundfarben umfasst, wobei jede Ausstrahlung durch wenigstens eine Licht mit der entsprechenden Grundlichtfrequenz ausstrahlende Elektrolumineszensdiode gebildet wird.

3. Vorrichtung zur Erfassung von Bildern gemäß einem der Ansprüche 1 bis 2, dadurch gekennzeichnet, daß die Elektrolumineszensdioden (3a-3c) in der pulsierenden Arbeitsweise übermodulierbare Dioden sind und daß das zyklische Verhältnis der Aktivierung der Dioden während einem Ausstrahlungszyklus klein ist, um durch eine Änderung der Leistung des ausgestrahlten Lichtes einen Ausgleich der ausgesandten Leistungsunterschiede des Lichtes mit verschiedenen Farben und der Unterschiede der Empfindlichkeiten gegen die Lichter mit verschiedenen Farben aus dem Sensor zu gestatten.

4. Vorrichtung nach einem der Ansprüche 2 oder 3, dadurch gekennzeichnet, daß jeder Ausstrahler (3a bis 3c) eine Vielzahl von Elektrolumineszensdioden aufweist, deren Anzahl gewählt wird, um die Unterschiede der Ausstrahlungssleistung und der Empfindlichkeit gegen die Lichter mit verschiedenen Farben aus dem Sensor (5) auszugleichen.

5. Vorrichtung nach einem der vorangehenden Ansprüche, dadurch gekennzeichnet, daß der einfarbige Lichtsensor (5) eine große Empfindlichkeit gegen Grundlichtfrequenzen der Beleuchtungsquelle (3) aufweist.

6. Vorrichtung nach einem der vorangehenden Ansprüche, dadurch gekennzeichnet, daß die Beleuchtungsquelle (3) eine durch optische Linsen, Spiegel oder optische Fasern gebildete optische Vorrichtung aufweist, welche Mittel (4) zur Änderung des Strahlungsdiagramms der Beleuchtungsquelle und zur Anpassung des Bildes des darzustellenden Gegenstandes an die Abmessungen des Lichtsensors (5) bilden.

7. Vorrichtung nach einem der Ansprüche 2 bis 5, dadurch gekennzeichnet, daß, um eine mehrere Lichtausstrahler aufweisende Lichtquelle zu bilden, die Halbleiterelemente (5) von wenigstens gewissen dieser Lichtausstrahler auf einem selben Substrat (16) angeordnet sind und daß eine gemeinsame optische Vorrichtung (18) diesen Lichtausstrahlern zugeordnet ist.

8. Vorrichtung nach einem der Ansprüche 1 bis 6, dadurch gekennzeichnet, daß sie einen Modul (8) zur Kontrolle der Arbeitsweise des Lichtsensors (5), welcher Modul die Aktivierung der Ausstrahler (3a bis 3c) der Beleuchtungsquelle (3) und einen in Seiten (7a, 7b-7n) organisierten Speichermodul steuert, welche jeweils die dem durch eine der Grundfarben der Lichtquelle (3) beleuchteten Gegenstand entsprechenden Ausgangssignale des Lichtsensors (5) in aufeinanderfolgenden Weise empfangen, und einen Umwandlungsmodul (9), der die Signale aus dem Lichtsensor (5) in mit dem Speichermodul (7) verträgliche Signale und die Signale des Speichermoduls (7) in mit den Normen zur Sichtbarmachung von mehrfarbigen Bildern verträgliche Signale (10) umwandelt, umfaßt.

9. Vorrichtung nach einem der vorangehenden Ansprüche, dadurch gekennzeichnet daß sie Betätigungsmittel (11) aufweist, um ein Bild einzufrieren.

10. Vorrichtung nach einem der vorangehenden Ansprüche, dadurch gekennzeichnet, daß die Lichtquelle (3), der Lichtsensor (5) und vorteilhaft die Mittel zur Verarbeitung der Signale in eine gegebenenfalls abgedichtete und sterilisierbare einzige Einheit kleiner Abmessungen eingegliedert sind.

11. Vorrichtung gemäß einer der Ansprüche 1 bis 9, dadurch gekennzeichnet, daß, um den Einfluß des Umgebungslichtes zu beseitigen, sie Mittel, um ein Bild des Gegenstandes in dem Umgebungslicht aufzunehmen und Mittel (33) zur Subtraktion dieses Bildes von den einfarbigen Bildern vor der Wiedergabe des Bildes in resultierenden Farben, um das Umgebungslicht in den einfarbigen Bildern zu beseitigen, aufweist.
